# EUROPÄISCHE PATENTSCHRIFT

(11) **EP 2 653 151 B1**
(45) Veröffentlichungstag und Bekanntmachung des Hinweises auf die Patenterteilung: **13.09.2017**
(21) Anmeldenummer: 13001837.7
(22) Anmeldetag: 09.04.2013
(51) Int. Cl.: A61K 8/92, A61K 8/96, A61K 8/02, A61K 8/19, A61K 8/20, A61Q 19/10

(54) **Stabile, einfach aus 2 Phasen herstellbare feste Ölbadesalzzubereitungen mit anwendungsbezogener Einsatzflexibilität**
Solid oil salt bath compositions easily to be prepared from 2 phases having an application related flexibility
Compositions solides de bain a l'huile et sel simplement preparable de 2 phases avec une flexibilite d'application

(30) Priorität: 18.04.2012 DE 102012007734
(43) Veröffentlichungstag der Anmeldung: 23.10.2013
(73) Patentinhaber: Merz Pharma GmbH & Co. KGaA, 60318 Frankfurt am Main (DE)
(72) Erfinder: Becker, Bärbel, 65719 Hofheim (DE); Ebinger, Jürgen, 65510 Hünstetten (DE); Berlekamp, Uwe, 48432 Rheine (DE)
(74) Vertreter: Müller, Claudia

(56) Entgegenhaltungen:
- EP-A2- 0 930 064
- WO-A1-2008/034549
- WO-A2-00/04867
- WO-A2-2011/006616
- CN-A- 101 152 123
- DATABASE GNPD [Online] MINTEL; August 2010 (2010-08), "Dead Sea Bath Salt", XP002734467, Database accession no. 1367960

## Beschreibung

### Gegenstand der Erfindung

Die vorliegende Erfindung betrifft kosmetische, dermatologische oder medizinische feste, im Wesentlichen wasserfreie (flüssige) Ölbadesalzzubereitungen zum flexiblen Einsatz in der Badeflotte, sowohl direkt als auch indirekt (gelöst), beim Duschen, als Fußbad u.ä. Diese bestehen aus einer festen Grundlage auf Basis eines Salzträgers (z. B. wie Mineral- und Meersalze), weiterhin umfassend ein oder mehrere wasserlösliche Tenside und ggf. Zusatz- und/oder Wirkstoffe; sowie einer flüssigen, ggf. Zusatz-und/oder Wirkstoffe umfassenden tensidhaltigen Ölgrundlage aus im Wesentlichen flüssigen Lipiden (Ölkomponente). In der Zubereitung sind mehr als 9 Gew.% (bezogen auf die Gesamtzubereitung) Ölkomponente, insbesondere mindestens 10 Gew.% und vorzugsweise mehr als 10 Gew.% Ölkomponente sowie höchstens 80 Gew.%, vorzugsweise weniger als 80 Gew.% Salz vorhanden. Die Gesamtmenge an Lipid(en) + Tensiden ist höher als 15 Gew.%. Die Zubereitungen liegen insgesamt als feste, schüttbare Produkte vor, die auch ohne Adsorber wie Aerosile, Dextrine, Kolloide stabil und einheitlich sind.

Je nach gewünschtem Einsatz können besondere Wirkstoffe inkorporiert werden wie Vitamine (Vitaminbad), Kräuter (Kräuterbad), Harnstoff (Pflegebad, hautstrukturierendes Bad, Peeling-Effekt), ätherische Öle (z. B. Entspannung, Beruhigung, Durchblutung) oder Kombinationen hiervon.

Derartige Zubereitungen (2-Phasen-Produkte) sind trotz der beiden zunächst in unterschiedlichen Aggregatzuständen vorliegenden Grundlagen fest, insbesondere auch schüttbar und weisen die Eigenschaften eines flüssigen Ölbades sowie eines festen Badesalzes derart auf, dass unerwarteter Weise auch ein verbesserter Effekt erzielt werden kann als bisher bei derartigen Einphasenprodukten nur mit weiteren Maßnahmen wie Liposomen oder Brausesatz möglich sind.

Die Zubereitungen werden hergestellt durch Zusammenmischen der festen Phase aus wasserlöslichem Tensid(en) TW, Trägersalz, ggf. wasserlöslichen Wirk- und/oder Zusatzstoffen. Die separat hergestellte Ölgrundlagenphase aus Lipid(en), lipid-(öl)löslichen Tensid(en) TO, ggf. lipidlöslichen Zusatz- und/oder Wirkstoffen wird sodann mit der festen Trägerphase ohne gesonderte Vermischung in geeignete Behältnisse vorzugsweise gleichzeitig eingefüllt. Es werden stabile, nicht emulgierte einheitliche Produkte aus festem Salz und nicht emulgierter Ölkomponente als Ölbadesalz erhalten, worin die Eigenschaften eines flüssigen Ölbades mit jenen eines festen Badezusatzes kombiniert sind, ohne dass die Stabilität der Inhaltsstoffe - wie z. B. durch die Zersetzung des Öls durch Salze - beeinträchtigt wird bzw. trotz des hohen Gesamttensid- und Lipidgehaltes kein Zusammenkleben erfolgt. Dennoch weisen die Zubereitungen einen überraschend guten Zusammenhalt auf, so dass sie anlässlich des Badevorgangs auch direkt auf der Haut per Hand angewendet werden können, z.B. als Peeling, zur besseren Durchblutung oder zur Verbesserung der Hautstruktur. Darüber hinaus werden höhere Pflege- und Wirkstoffeffekte erzielt, wie sie bisher nur durch bestimmte Maßnahmen wie Liposomale Strukturen, Brausesatz, Proteosomale Anteile (aus Liposomenbildner und Proteinen, s.u.) erreicht wurden. Die anmeldungsgemäßen Zubereitungen können vorzugsweise portioniert vorliegen, wodurch eine einfache und sichere Handhabung gewährleistet ist.

### Hintergrund der Erfindung

Balneologische Zusätze zur Anwendung in der Wanne oder der Dusche sind seit langem bekannt. Dabei werden wasserfreie, insbesondere ölhaltige Produkte von wasserhaltigen Produkten unterschieden, welche je nach Zusatzstoffen unterschiedliche Konsistenzen von flüssig bis gelartig aufweisen können. Sie enthalten vor allem Tenside zur Reinigung der Haut sowie vorzugsweise lipidische Substanzen, um den durch die Anwendung von Wasser und Tensiden entfernten Lipidfilm wiederaufzubauen. Bevorzugt werden hier auch ätherische Öle / Extrakte je nach beabsichtigter Wirkung eingesetzt. Besonders geeignet sind auch nicht wässrige Formulierungen, enthaltend liposomenbildende Substanzen. Hierdurch soll ein verbesserter Wirkstofftransport und damit eine höhere Wirksamkeit bei der Anwendung erfolgen. Ein Problem ist dabei die Stabilität der Liposomen, welche durch unterschiedlichste Einflüsse wie Elektrolytgehalt, Emulgatoren, Wasser u. ä. eingeschränkt sein kann. So werden gemäß der DE 42 05 548 A1 bestimmte stabilisierende wasserdispergierbare öllösliche polyoxyethylierte Tenside mit einem HLB- Wert von 6-13 zusammen mit Vesikelbildnern wie Phospholipiden in einer Ölgrundlage beschrieben, welche erst bei Kontakt mit Wasser, z. B. Eingießen in die Badeflotte, liposomale Strukturen ausbilden. Dadurch kann eine protrahierte Wirkstofffreisetzung mit Depoteffekt erzielt werden.

Gemäß der EP 0930 064 B1 sind feste Badezusätze in Form von Pulvern oder Granulaten bekannt, welche neben einem Brausesatz einen Mineralsalzgehalt, insbesondere feste Lipid- Komponenten wie hydrierte Öle oder Glyceride, ferner W/O und ggf. O/W-Tenside sowie vesikelbildende Komponenten ggf. zusammen mit geeigneten Zusatzstoffen aufweisen können. Dabei wurde das Problem gelöst, die aus flüssigen Badepräparaten bekannten Tensid- Lipid- Liposomen-Kombinationen auf feste sprudelnde Badetabletten zu übertragen, obgleich derartige Komprimate nur äußerst geringe Flüssigkeits- und Fettanteile enthalten können, um stabil zu bleiben. Überraschender Weise können damit in der Badeflotte trotz des hohen Salzgehaltes (bis zu 80%) liposomale Strukturen ausgebildet werden. Auch hiermit ist ein effektiver Wirkstofftransport vor allem lipidlöslicher Stoffe möglich.

Die WO 2011/006616 betrifft balneologische Zubereitungen, insbesondere Bade- und Duschzubereitungen, die als fließfähiges oder flüssiges Prämix-Konzentrat neben natürlichen und/oder synthetischen Lipiden, Tensiden liposomenbildende Phospholipide in Kombination mit Proteinen (MW bis 30000 Da) aufweisen. Letztere beiden Komponenten bilden bei Kontakt mit einem Überschuss an Wasser unmittelbar hautaktive Proteoliposomen (Proteosomen) mit katalytischer Aktivität für einen verbesserten Wirkstofftransport in die Haut. Sie können direkt oder in verdünnten oder festen Anwendungsformen eingesetzt werden. In festen Zubereitungen beträgt der Ölanteil maximal 9%, der Tensidgehalt 6%, der Salzgehalt 82% und mehr.

Die WO 2010/022873 A2 beschreibt isometrische Agglomerate, insbesondere Badeperlen aus 80 to 97 Gew.% eines festen Trägers wie Kollagenhydrolysat, Gelatine oder Gelatinehydrolysat, Cellulose, Kohlenhydrate, Stärke oder Harnstoff mit bis zu 10% Lipiden, vorzugsweise Mischungen aus festen und flüssigen Lipiden, und bis zu 11 Gew.% Tensiden; sowie liposomenbildenden Substanzen. Zum einen sind die Träger hier nicht salzartiger Natur, sondern kolloidal und weisen daher auch gute Adsorptionseigenschaften auf, zum anderen werden liposomale Strukturen zur Wirkverbesserung eingesetzt.

Gemäß der DE 10 2008 010 921 A1 werden Badezubereitungen beschrieben, welche 4 unterschiedliche Phasen aufweisen, die dann jeweils separat und voneinander getrennt in einer gemeinsamen Umverpackung bereitgestellt werden. Der Anwender ist angewiesen, die einzelnen 4 Phasen in einer vorgegebenen Reihenfolge in eine vorbestimmte Wassermenge einzubringen, wobei eine Wirkstoffgrundlage mit Tensiden, eine Gelgrundlage, eine Stabilisierungsgrundlage zur Viskositätseinstellung und ein Reinigungspaket getrennt vorliegen müssen.

Die DE 10 2011 009 798 A1 betrifft balneologische lipidhaltige Badezusätze mit einem Probiotikaanteil. Die Zusätze sind wasserfrei und entweder fest auf Basis von Mineralsalzen (Badesalz) oder flüssig auf Basis von Ölen (Ölbad). Es liegen jeweils einphasige Produkte vor, aus denen die Probiotischen Wirkstoffe abgegeben werden. Die EP 1 157 940 B1 beschreibt eine Kräuterpackung zur Herstellung eines Heil-und Pflegebades, wobei die Kräuter zusammen mit Molkepulver, Salz, Rückfettendem Wirkstoff, Farbstoffen in einem Beutel als Mischung vorhanden sind.

Es wäre jedoch wünschenswert, wenn eine Zubereitung bereitgestellt werden könnte, welche die Vorzüge einer leicht zu handhabenden festen Darreichungsform auf Salzgrundlage sowie einer lipidhaltigen Ölzubereitung vereinigt, und insbesondere höhere Mengen an letzterer aufnehmen kann, ohne dass die Stabilität insbesondere der Lipidgrundlage beeinträchtigt wird. Eine solche stabile Zubereitung sollte ferner eine anwendungsorientierte Einsatzflexibilität aufweisen. Beispielsweise sind derzeit keine festen Salz- oder Badetabletten-Zubereitungen mit höherer Ölgrundlage bekannt, welche z.B. 10 Gew. % Harnstoff als Pflegestoff neben Salzen umfassen. Ferner sollten die Zubereitungen auf einfache Weise allgemein - z. B. in der Wanne oder auch im Duschbad - gehandhabt werden können und gleichzeitig mit dem Reinigungsvorgang die Haut - ohne dass dafür spezielle Wirkstoffaktivatoren/-promotoren/ Penetrationsförderer (wie Liposomen, Proteosomen, CO₂) erforderlich wären - mit anwendungsspezifischen Wirk- und Pflegestoffen versorgt werden können, wobei auf aufwendige Herstellungsverfahren sowie expensive, vor allem auch nicht natürliche Grundstoffe verzichtet werden kann.

### Aufgabe der Erfindung

Der vorliegenden Erfindung liegt die Aufgabe zugrunde, eine einfach zu handhabende balneologische feste, stabile und einsatzflexible Zubereitung zur Verfügung zu stellen, mit welcher die Haut anlässlich des Reinigungsvorgangs wie z. B. beim Baden oder Duschen, je nach Indikation ausreichend mit anwendungsspezifischen Wirkstoffen, wie z.B. Hautgesunden Vitaminen (sowohl wasserlöslich als auch fettlöslich), Kräutern, Pflegestoffen auch ohne dass zusätzliche Wirkpromotoren zwingend erforderlich sind, versorgt werden kann. Die Zubereitung soll insbesondere einfach einzusetzen sein. Wünschenswert wäre auch die Anwendung natürlicher Grundstoffe sowie der Verzicht auf aufwendige Herstellungsverfahren sowie expensive Ausgangssubstanzen/Herstellungsverfahren.

### Lösung der Aufgabe

Diese Aufgabe wird erfindungsgemäß dadurch gelöst, dass man eine im Wesentlichen wasserfreie feste Ölbadesalzzubereitung aus 2 nicht emulgierten Phasen (fest- flüssig) bereitstellt, welche zusammengesetzt ist aus einem festen, ggf. Zusatz- und/oder Wirkstoffe umfassenden tensidhaltigen Träger, ausgewählt aus Salzen, insbesondere Mineralsalzen, Meersalzen, Steinsalzen, Siedesalzen, Totes Meer Salz (jeweils vor allem ohne Brausesatz aus Carbonaten/Säuren), und einer flüssigen, ggf. Zusatz-und/oder Wirkstoffe umfassenden tensidhaltigen Ölgrundlage aus im Wesentlichen flüssigen Lipiden (Ölen). In derartigen festen Ölzubereitungen kann der Gesamttensidgehalt gleich, insbesondere auch niedriger als der Gehalt an Lipiden sein. Insbesondere ist die Gesamtmenge an Lipid(en) + Tensid(en) höher als 15 Gew.%. Überraschenderweise liegen die erfindungsgemäßen Zubereitungen, obgleich es sich um Produkte aus Ausgangsmaterialien verschiedener Aggregatzustände, nämlich flüssiger Ölgrundlage neben fester Trägerphase handelt, in fester, insbesondere körniger Form vor. Sie sind schüttbar und stabil, obgleich Adsorber, wie sie normalerweise in derartigen Produkten erforderlich sind (z. B. Aerosile, Kolloide, Dextrine, oder Cellulosederivate), hier nicht enthalten sind (sein müssen), während andererseits die Ölphase flüssig ist. Überraschenderweise weisen die erfindungsgemäßen Zubereitungen dennoch einen guten Zusammenhalt auf. Sie können daher auch per Hand vor oder während des Badevorgangs auf die Haut aufgetragen werden, wodurch, insbesondere auch durch Einreiben der Zubereitung, je nach Ziel weitere Effekte möglich sind wie Peeling, verbesserte Hautdurchblutung, verbesserte Hautstruktur. Die erfindungsgemäßen Zubereitungen sind daher in mehrfacher Hinsicht gut handhabbar bzw. einsatzflexibel.

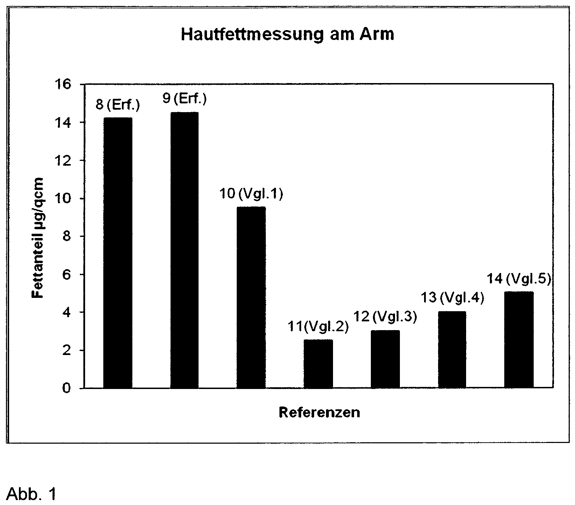

Ferner ist es auch nicht erforderlich, dass bei der Herstellung erfindungsgemäßer Zubereitungen gesonderte Emulgierschritte vorgenommen werden. Darüber hinaus kann durch den Anteil an flüssigen Lipiden eine hautverbessernde Wirkung gegenüber Produkten mit festen Lipiden festgestellt werden, wie aus dem experimentellen Beispiel 15 sowie der dazugehörigen Abbildung 1 ersichtlich ist und dort näher erläutert wird. Dabei können auch eventuell durch den Salzanteil auslaugende Effekte reduziert bzw. vermieden werden. Je nach gewünschtem Einsatz können besondere Wirkstoffe inkorporiert werden wie Vitamine (Vitaminbad), Kräuter (Kräuterbad), Harnstoff (Pflegebad) oder Kombinationen hiervon.

Durch die lokale Kombination der jeweiligen nicht miteinander emulgierten Phasen ist es überraschenderweise möglich, auch Wirkstoffkombinationen wie wasser- und ölllösliche Vitamine gleichzeitig oder auch hohe Pflegewirkstoffmengen wie z. B. 10 und mehr Gew.% Harnstoff zu inkorporieren.

Das oder die Tenside im festen Träger sind dabei wasserlöslich, das oder die Tenside der Ölgrundlage sind insbesondere öllöslich. Insbesondere umfasst das wasserlösliche Tensid (TW) mindestens ein Mittel, welches ausgewählt ist aus anionischen C₁₂₋₂₂-Fettalkoholethersulfaten, C₁₂₋₂₂₋- Sulfo-Succinaten wie z.B. Disodium Lauryl- Sulfosuccinat und/oder C₁₂₋₂₂₋-Fettsäureamidsulfaten, während das öllösliche Tensid (TO) vor allem ein polyoxylierter Fettalkoholether, ein polyoxyethylierter Fettsäureester, ein Zuckerester und/oder Zuckerether von jeweils C₁₂₋₂₂₋Fettsäuren oder C₈₋₂₂- Fettalkoholen ist.

Die erfindungsgemäße feste Ölbadesalzzubereitung besteht vor allem aus mehr als 10, insbesondere 11 bis 35 Gew.% flüssiger Ölgrundlage. Dabei umfasst die Ölgrundlage hier Ölkomponente/n (Lipide), ein oder mehrere Tenside (TO) sowie ggf. Zusatzstoffe/Wirkstoffe. Dementsprechend umfasst die Gesamtzubereitung (feste Ölbadesalzzubereitung) mehr als 9, vor allem mindestens 10 Gew. %, insbesondere mehr als 10 Gew.% Ölkomponenten sowie 65 bis weniger als 90 Gew.% feste Trägergrundlage, wobei diese hier das Salz, ein oder mehrere Tenside (TW) und ggf. Zusatz-und/oder-Wirkstoffe umfasst, mit der Maßgabe, dass höchstens 80 Gew.% Salz sowie mehr als 9 Gew.%, insbesondere mindestens 10 Gew. % Ölkomponente umfasst sind, und die Gesamtmenge an Lipid(en) + Tensiden in der Gesamtzubereitung mehr als 15 Gew.% beträgt. Das Salz ist vorzugsweise ausgewählt aus Mineralsalzen, z. B. Natriumchlorid, Natriumsulfat, Totes Meersalz, Meersalz, Steinsalz, Siedesalz u.ä. oder Mischungen hiervon.

Die Ölgrundlage weist vor allem ein oder mehrere natürliche flüssige Öle auf. Die Zubereitung umfasst vorzugsweise Wirkstoffe wie vor allem Vitamine, Kräuter, PflegeStoffe. Als Vitamine werden vorzugsweise eingesetzt: Vitamin C wie Vitamin-C- Palmitat, Calciumascorbat, Vitamin E (z. B. Acetat; -Palmitat, -Phosphat, -Succinat), Vitamin- A wie Retinol- acetat, -proprionat,- palmitiat, -phosphat, Vitamin B7/H (Biotin), Pantothensäure (Vitamin B5 wie Calciumpantothenat), Vitamin B1 (Thiamin wie Thiaminnitrat), Vitamin B2 (Riboflavin, Riboflavin-5-Phosphat-Natriumsalz), Vitamin B3 (Niacin), Vitamin B6 (Pyridoxin wie Pyridoxinhydrochlorid), ggf. Vitamin B12 (Cyanocobalamin), bzw. Mischungen hiervon, vor allem von 3 oder 4 oder mehreren hiervon. Eine ganz besonders geeignete Multivitamin-Gruppe umfasst Vitamin A, wie vor allem Retinolpalmitat, ein oder mehrere Vertreter der Vitamin B-Gruppe wie Biotin (B7), Pantothensäure (B5) sowie Vitamin C- Palmitat.

Als Kräuter werden vor allem Kamille, Schachtelhalm, Heublume, Thymian, Lavendel, Rosmarin (Blätter oder Blüten) oder Mischungen hiervon eingesetzt.

Als Pflegstoffe eignen sich insbesondere Harnstoff, Lecithine und / oder C₈-C₂₂- Alkylglucosid-Fettsäureglycerylester wie Lamesoft, Softigen (Coco Glucoside Glyceryl Oleat).

Bevorzugt umfasst die Ölgrundlage als Ölkomponenten 70 bis 90 Gew.% Lipide (vor allem flüssige Lipide = Öle), 1 bis 20 Gew.%, vor allem 2 bis 15 Gew.% öllösliche Tenside (TO), 1,5 bis 7 Gew.% darin lösliche Wirkstoffe wie vor allem Vitamine, 0 bis 10, vor allem 0,1 bis 9 Gew.% Zusatzstoffe sowie weniger als 1 % Wasser.

Die feste Trägergrundlage der erfindungsgemäßen Zubereitung umfasst vor allem 70 bis 90 Gew.% eines oder mehrerer Mineralsalze wie Chloride, Sulfate von Alkali- und Erdalkalimetallen (NaCl, CaSO₄, MgSO₄, KBr, NaSO₄), sowie ein oder mehreren wasserlösliche anionische Tenside (0,5 bis 10, vor allem 1 bis 10, insbesondere 1,5 bis 7 Gew.%), sowie 0,5 bis 15, vor allem 1 bis 8 Gew.% feste Wirkstoffe, insbesondere wasserlösliche Kräuter wie beschrieben, Harnstoff (z. B. bis zu 12 Gew.%) und/oder ggf. Zusatzstoffe (z. B. 0,1 bis 10 Gew.%,).

Überraschender Weise hat sich gezeigt, dass trotz der relativ kurzen Kontaktzeit der Haut im Bade - insbesondere im Vergleich zur Applizierungszeit einer Creme - eine hohe Versorgung mit Lipiden und ggf. Wirkstoffen wie z. B. Vitaminen, Kräutern, Pflegestoffen erfolgen kann, wobei diese nicht lokal begrenzt, sondern ganzheitlich, einschließlich der Schleimhaut, erfolgt. Ohne an eine Theorie gebunden zu sein, wird angenommen, dass die Haut aufgrund der Salzwirkung einer gewissen Quellung unterliegt und im Zusammenwirken mit der höheren Thermik diese aufnahmefähiger wird für das in höheren Mengen vorhandene flüssige Lipid und ggf. die verfügbar gemachten Wirkstoffe. Ein salzbedingtes Auslaugen der Haut kann somit vermieden werden. Das Hautareal wird insgesamt flächendeckend erreicht, da die flüssigen Lipide - anders als in bisher beschriebenen Produkten mit festen Lipiden - in der Zubereitung separat vorliegen und dadurch leicht verteilt werden können. Somit kann ein gleicher bzw. besserer Lipidstatus wie durch ein reines Ölbad erzielt werden, ohne dass z. B. Liposomen dazu erforderlich wären. Insbesondere sind Adsorber wie Kolloide, Proteine (wie z. B. nachfolgend beschrieben), Dextrine, Aerosile nicht erforderlich. Die Stabilität der Produkte kann durch die beschriebenen Verhältnisse der Inhaltsstoffe zueinander überraschender Weise erhalten bleiben. Darüber hinaus wird mittels der eingesetzten Tenside auch ein milder Reinigungseffekt erzielt. Je nach Anteilen an Lipid/Tensid/Wirkstoff kann somit eine spezielle Wirkung gesteuert werden. Auch dadurch weisen die Zubereitungen eine Einsatzflexibilität auf.

Die erfindungsgemäßen Zubereitungen sind trotz der 2- Phasigkeit, insbesondere körnig, schüttbar und können aufgrund des guten Zusammenhalts auch direkt per Hand auf der Haut angewendet werden. Sie sind daher einfach und besonders effektiv zu handhaben. Die feste Zubereitung kann vorzugsweise in Einzelgebinde (Sachets) abgepackt und als Einmalanwendung eingesetzt werden. Im Falle von Kräutern oder zur Anwendung in der Dusche können die Gebinde auch perforiert sein, z. B. in Form von flexiblen Kissen, Schlauchbehältnissen u.ä. und aus geeigneten Materialien wie Baumwolle, Gaze, oder aus anderem für derartige Zwecke geeignetem und bekanntem textilem Kunststoffgewebe bestehen. Diese perforierten Gebinde werden dann zweckmäßiger Weise in Sachets verpackt. Andererseits können auch größere Mengen für mehrere Anwendungen in einer Packung enthalten sein, aus der dann jeweils die gewünschte Menge an Ölbadesalzzubereitung leicht entnommen werden kann.

,2-Phasisgkeit' bedeutet anmeldungsgemäß eine an sich schüttbare Zubereitung, worin eine feste Trägerphase und eine flüssige Ölphase nebeneinander (nicht miteinander gemischt) vorliegen, ohne dass diese Phasen miteinander durchmischt/emulgiert (wie bei Aufeinanderauftragung und Mischung) sind.

### Abbildung

Abbildung 1 zeigt die graphische Darstellung der nach der Sebumeter-Methode (Hautfettmessung) erhaltenen Meßwerte von erfindungsgemäßen Zubereitungen (Beispiele 8, 9) und Vergleichszubereitungen (Beispiele 10-14) hinsichtlich des Fettanteils untersuchter Haut nach dem Bade. Wie ersichtlich werden mit erfindungsgemäßen Zubereitungen bessere Ergebnisse erzielt als mit bekannten Vergleichs-Ölbadezubereitungen.

### Nähere Erläuterung der Erfindung

Die vorliegende Erfindung betrifft feste Ölbadzubereitungen sowohl für kosmetische, als auch für dermatologische oder medizinische Indikationen mit anwendungsbezogener Wirk- und Einsatzflexibilität und einfacher Handhabung sowie hoher Stabilität trotz der Anwesenheit hoher Mengen an flüssigen Komponenten in nicht absorptiven Feststoffen in einem einzigen Produkt.

### Beschreibung der Inhaltsstoffe

### 1. Lipide (Ölkomponente)

Die festen Zubereitungen vorliegender Erfindung umfassen zunächst ein oder mehrere Lipide in einer Menge von 10 bis 35 Gew.%, bezogen auf das Gesamtgewicht der Zubereitung. Die Lipide sind ausgewählt aus natürlichen flüssigen (ungesättigten) Ölen. Weiterhin sind flüssige C₈₋₂₂₋- Fettalkohole wie Oleylalkohol, Octyldodecanol, Cetyl-/stearylalkohol geeignet, wenn sie mit einem oder mehreren natürlichen Ölen eingesetzt werden.

Bevorzugte Lipide sind ausgewählt aus flüssigen Lipiden (natürlichen Ölen) der Gruppe, umfassend:
Olivenöl, Sonnenblumen-, Sojaöl, Pfirsichkern-, Aprikosenkern-, Traubenkernöl, Ricinusöl, Erdnuß-, Mandel-, Nerz-, Weizenkeim-, Sesam- Distel- Mandel-, Avocadoöl, Shea Butter oder Illipé Butter; Natürliche flüssige Wachse, z.B. Jojobaöl, Kokosnussöl, Borretschöl, Maiskeimöl, Walnussöl, Palmöl, Macadamianussöl, Palmkernöl, Haselnussöl, Hagebuttenkernöl, Baumwollsamenöl, Papayaöl oder Mischungen hiervon.

Besonders bevorzugte flüssige Lipide sind Pflanzenöle, ausgewählt aus Sojaöl, Sonnenblumenöl, Weizenkeimöl, Olivenöl oder Mischungen hiervon.

Die Lipide (Ölkomponenten) sind in erfindungsgemäßen Ölgrundlagen vorzugsweise in einer Gesamtmenge von 35 bis 95 Gew.%, insbesondere 50 bis 90 Gew.% (flüssige, wasserfreie Grundlagen) vorhanden, wobei in der Endzubereitung (festes Ölbad) dann vorzugsweise mindestens 10 Gew.% flüssiges Lipid (Öl) vorhanden sein müssen.

In einer besonderen Ausführungsform können auch natürliche Öle im Gemisch mit höheren Fettalkoholen als Lipide enthalten sein, wobei der Alkylteil letzterer ausgewählt sein kann aus C₈-C₂₂-, insbesondere C₁₄-C₂₀-Alkyl. Hierzu gehören z.B. 2-Octyldodecanol, Oleylalkohol oder Mischungen hiervon. Die Lipide gemäß vorliegender Erfindung werden besonders bevorzugt ausgewählt aus der Gruppe, umfassend natürliche flüssige Öle oder Mischungen hiervon, insbesondere solche mit einem hohen Gehalt an Ölsäure und (+) Linolsäure, vor allem solche, deren Gesamtanteil (∑) an Ölsäure + Linolsäure mindestens 50 %, insbesondere mehr als 60 % und ganz besonders bevorzugt mindestens 75 % beträgt. (Angaben zu Ölsäure- Linolsäuregehalt oben beschriebener Öle können z. B. der DAZ, 150. Jahrgang, 02.09.2010, Nr. 35, Seite 3925-3934 entnommen werden).

Es ist ferner bevorzugt, wenn in einer Lipidmischung mindestens eine Substanz mit einem Gesamtanteil (∑) von Ölsäure + Linolsäure von wenigstens 75 Gew.%, bevorzugt wenigstens 80 Gew.% vorhanden ist.

Für erfindungsgemäße Zubereitungen geeignete flüssige Lipide sind daher: Aprikosenkernöl, Avocadoöl, Borretschsamenöl, Distelöl, Erdnussöl, Haselnussöl, Kürbiskernöl, Maiskeimöl, Mandelöl, Marulaöl, Macademianußöl, Mohnöl, Olivenöl, Palmöl, Pinienkernöl, Reiskeimöl, Sanddornöl, Sesamöl, Sojaöl, Sonnenblumenöl, Traubenkernöl, Walnussöl, Weizenkeimöl, Erdnußöl, Distelöl, oder Mischungen dieser Öle.

Hydrierte Derivate dieser Öle oder andere feste Lipide werden bevorzugt nicht eingesetzt, so dass die Zubereitung im Wesentlichen frei von solchen festen Lipiden ist. Ganz besonders bevorzugte flüssige Lipide sind Pflanzenöle, ausgewählt aus Mandelöl, Avocadoöl, Marulaöl, Aprikosenkernöl, Haselnussöl, Sanddornöl, Sojaöl oder Mischungen hiervon.

In einer anderen bevorzugten Ausführungsform umfassen anmeldungsgemäße Zubereitungen wenigstens ein flüssiges Lipid, ausgewählt aus Sonnenblumen-, Sojaöl, Olivenöl, Pfirsichkern-, Aprikosenkern-, Traubenkern-, Erdnuß-, Mandel-, Nerz-, Weizenkeim-, Sesam- Distel- Mandel-, Avocadoöl, Palmöl, Pinienkernöl, Reiskeimöl, Macadamianussöl, Palmkernöl, Papayaöl, Sanddornöl (mit jeweils einem Ölsäure-Linolsäuregesamtgehalt (∑) von mindestens 50 Gew. %), zusammen mit einem oder mehreren Pflegestoffen, vorzugsweise ausgewählt aus (auch als Vesikelbildner bekannten) Lecithinen, Phospholipiden, Sphingosinen, Cerebrosiden, Ceramiden oder Gemischen hiervon wie nachfolgend beschrieben.

In anderen geeigneten Ausführungsformen werden ein oder mehrere Öle wie vorgenannt, vor allem Sonnenblumen-, Sojaöl, Pfirsichkern-, Aprikosenkern-, Traubenkern-, Ricinus-, Erdnuß-, Mandel-, Nerz-, Weizenkeim-, Sesam- Distel- Mandel-, Avocadoöl, Palmöl, Macadamianussöl, Palmkernöl, Papayaöl, kombiniert mit einem oder mehreren C₈-C₂₂- Fettalkoholen wie insbesondere Oleylalkohol eingesetzt.

### 2. Tenside

Die festen Ölbadezubereitungen umfassen weiterhin Tenside, und zwar wasserlösliche anionische Tenside sowie öllösliche nichtionische Tenside.

Als wasserlösliche Tenside werden vorzugsweise anionische Substanzen ausgewählt, wie z. B. C₈₋₂₂-Alkylsulfate bzw. C₈₋₂₂ -Alkylethersulfate bzw. deren Alkali- insbesondere Natriumsalze wie Natriumlaurylsulfat, Natriumlaurylethersulfat oder auch vor allem aus (Alkyl)- C₁₂ -C₂₂- Fettalkohol- Aminosäureester- Verbindungen, insbesondere eines Alkalisalzes hiervon, wobei die Aminosäuren ausgewählt sind aus Glycin, Taurin, wie Oleyltaurat, Lauryltaurat, Natriummethyloleyltaurat u.ä.. Diese können als Salze eingesetzt werden wie z. B. Alkali-/Erdalkalisalze (Na-/K-Ca-, Mg)-Chloride, - Sulfate,- Phosphate. Ferner geeignet sind auch C₁₂₋₂₂-Sulfo-Succinate wie z.B. Disodium Lauryl Sulfosuccinat (im Handel erhältlich z. B. als Plantaton® SUS).

Als öllösliche Tenside werden vorzugsweise nichtionische, insbesondere polyoxylierte Fettalkohole und Fettsäureester eingesetzt. Hierzu gehören: niedrigpolyethoxylierte oder -propoxylierte bzw. auch gemischt niedrigpolyethoxy/propoxylierte C₈₋₂₂-Fettalkohole mit einem Ethoxylierungsgrad und/oder Propoxylierungsgrad (EO/-PO-Grad) von 1-5 EO. Bevorzugt sind niedrigpolyoxylierte C₈₋₂₂- Fettalkohole mit einem EO Grad von 1-5. Dazu zählen vor allem Macrogol-(Polyethylenglykol)laurylether mit 1 bis 4 EO-Einheiten wie Laureth 2, Laureth-3, Laureth-4 oder Mischungen hiervon. Weiterhin geeignet sind höher polyoxyethylierte und/oder -propoxylierte- C₈₋₂₂-Fettalkohole (vor allem Alkyl-C₁₀-C₁₈-Fettalkoholalkoxylate) oder derartige ethoxylierte und/oder propoxylierte C₁₂₋₂₂-Fettsäuren mit Ethoxylierungs- und/oder Propoxylierungsgraden von 8-25 oder Mischungen hiervon wie: Polyoxypropylenstearylether oder, Polyoxyethylensorbitanmonooleat, Polyoxyethylensorbitanmono-stearat - oder - palmitat, Polyoxyethylenstearylether, Polyoxyethylenoleyether, Polyoxyethylenoxypropylenmonostearat, Polyoxyethylenfettalkoholether, Polyoxyethylenmonostearat u.ä.. Insbesondere bevorzugt sind hier ethoxylierte C₁₂₋₂₂- Fettsäuren oder ethoxylierte C₈₋₂₂- Fettalkohole mit Ethoxylierungsgraden von 10-20.

Weiterhin geeignet als öllösliche Tenside sind auch Zuckerester oder Zuckerether von C₁₂₋₂₂- Fettsäuren, C₁₂₋₂₂ -Fettsäureglycerylestern oder C₈₋₂₂-Fettalkoholen. Dazu zählen insbesondere Zuckerester von C₁₂-C₁₈- gesättigten, ungesättigten, partial gesättigten Fettsäuren; polyoxyethylierte und/oder polyoxypropylierte Zuckerester von C₁₂-C₁₈- Fettsäuren, wobei der EO bzw. PO Grad 8-25 beträgt. Zu den Zuckerethern gehören solche von C₈-C₂₀- gesättigten, ungesättigten, partial gesättigten Fettalkoholen; oder von polyoxyethylierten und/oder polyoxypropylierten Zuckerethern von diesen C₈-C₂₀- Fettalkoholen. Der EO- bzw. PO Grad liegt zwischen 8 und 25. Als Zucker sind insbesondere Glukose, Methylglucose, Saccharose geeignet und als Säuren oder Alkohole die C₁₆-C₁₈- Fettsäuren /-Alkohole. Bevorzugt sind monooxylierte Produkte (polyethoxylierte oder polypropoxylierte Produkte) und darunter insbesondere ethoxylierte Produkte. Die Säure- bzw. Alkoholkomponenten leiten sich vor allem von aliphatischen Carbonsäuren/-alkoholen ab. Hierzu gehören insbesondere Caprylsäure, Caprinsäure, Laurinsäure, Myristinsäure, Palmitinsäure, Stearinsäure, Ölsäure und auch Mischungen hiervon bzw. derartigen Fettalkoholen (Oleylalkohol, Laurylakohol Stearylalkohol usw.).

Die Tenside (TW+TO) sind insgesamt in einer Menge von 3 bis 20 Gew.%, bezogen auf die gesamte feste Ölbadbereitung vorhanden, insgesamt derart, dass die Summe an Lipid+Tensid mehr als 15, vorzugsweise mindestens 16 Gew.% beträgt, wobei die Menge an Ölkomponente (Lipid) mindestens 10 Gew.% (Gew.%- Angaben bezogen auf die feste Gesamtzusammensetzung) beträgt. Die Tenside liegen vorzugsweise im festen Träger in einer Menge von 0,5 bis 5 Gew.%, vor allem als wasserlösliches Produkt, und in der Ölgrundlage in einer Menge von 3 bis 13 Gew.% als öllösliches Produkt vor.

Ein besonders geeignetes Zuckertensid ist Coco Glucoside Glyceryl Oleat.

Besonders bevorzugte TO-Tenside werden ausgewählt aus niedrig- polyethoxylierten oder niedrigpolypropoxylierten bzw. auch gemischt oxylierten C₁₂₋₁₈- Fettalkoholen mit einem Ethoxylierungsgrad und/oder Propoxylierungsgrad (EO/PO-Grad) von 1-5, insbesondere mit einem Ethoxylierungsgrad (EO- Grad) von 2-4 wie vor allem derartige Laurylether- Komponenten, insbesondere in Kombination mit anionischen TW- Tensiden wie C₈₋₂₂ -Alkylsulfaten, C₈₋₂₂ - Alkylethersulfaten sowie C₁₂-C₁₈- Fettalkoholen-Tauraten, insbesondere Natrium-Methyl-Oleyltaurat, oder auch Disodiumlaurylsuccinat.

Weiterhin bevorzugte TO- Tenside sind oxylierte C₁₂₋₁₈-Fettsäureester mit einem Ethoxylierungsgrad und/oder Propoxylierungsgrad (EO/PO-Grad) von 1-5 oder derartige Amide, wobei monooxylierte Produkte und darunter vor allem auch Ethoxylierungsprodukte bevorzugt sind wie vor allem: Polyoxyethylenlaurylether mit 1 bis 4 EO-Einheiten, mittlerer Kettenlänge wie Polyoxyethylen-Oleylether, Polyoxyethylen-Glycerylcocoat, Cocosfettsäurediethanolamid, Polyoxyethylenglyceroltrioleat, Polyoxyethylensorbitantristearat, Polyoxypropylenstearylether. Insbesondere bevorzugt sind hier Polyoxyethylenlaurylether mit 1 bis 4 EO-Einheiten, Polyoxyethylen-Glycerolcocoat, Cocosfettsäurediethanolamid oder deren Mischungen, in Kombination mit einem oder mehreren o.g. anionischen TW-Tensiden.

### 3. Feste Träger

Hierzu zählen die für derartige balneologische Zubereitungen bekannten Salze wie Mineralsalze, insbesondere Salze aus den Kationen Natrium, Kalium, Magnesium, Calcium, Eisen, Ammonium oder Mangan und den Anionen Chlorid, Fluorid, Sulfat oder Nitrat als solche oder als Mischungen eingesetzt (Natrium-/Magnesium-/Calciumchlorid, Na-(Mg-/Ca-bromid,-sulfat, Kaliumfluorid, u.ä.). Ferner können auch andere aus dem Thermalbadmileu bekannte Salze, wie z.B. bei den Kationen Lithium, Aluminium, Strontium, und bei den Anionen Iodid, Bromid, Thiosulfat, eingesetzt werden. Hierdurch können thermalbadanaloge Bedingungen erzielt werden.

Auch Meersalz, Pfannensiedesalz, Totes Meersalz, Steinsalz oder Siedesalz oder geeignete Mischungen hiervon können in den erfindungsgemäßen Zubereitungen als Salzgrundlage eingesetzt werden. Vorzugsweise werden ausgewählt: Natriumsulfat, Meersalz, Natriumchlorid, Natriumbromid, Magnesiumsulfat, Calciumchlorid, Calciumsulfat, Totes Meersalz, Siedesalz oder Mischungen hiervon.

Wesentlich für die Gesamtzubereitung ist ein Gesamtanteil an Salz von höchstens 80 Gew.%, vorzugsweise 69 bis 78 Gew.%.

Ein Brausesatz wie bisher für diese Produkte beschrieben aus Carbonatsalzen wie Calcium-/Magnesiumcarbonat und entsprechenden Mengen an Säuren wie Zitronensäure wird hier vorzugsweise nicht eingesetzt.

### 4. Zusatz-und Wirkstoffe

Zusatz- und Wirkstoffe können in Mengen von 0 bis 20 Gew.% oder 0 bis 10 Gew.%, vor allem 0,1 bis 15 Gew.%, insbesondere 0,1 bis 10 Gew.% vorliegen.

### 4. 1.Wirkstoffe

Wirkstoffe (z. B. in Mengen von 0,1 bis 15, vorzugsweise 0,2 bis 10 und vor allem 0,2 bis 8 Gew. %, bezogen auf die Gesamtzubereitung) sind je nach Aggregatzustand bzw. Löslichkeit entweder in der festen (wasserlöslichen) Phase oder der flüssigen Ölphase eingearbeitet und können daher einzeln oder auch kombiniert eingesetzt werden. Insbesondere können somit wasserlösliche Wirkstoffe mit lipid(öl)löslichen Wirkstoffen sowohl in höheren Mengen als auch in einer einzigen festen Ölbadsalzzubereitung kombiniert werden, wobei mit der Gesamtzubereitung mit den flüssigen Lipiden und deren leichter Lösbarkeit eine gleichmäßige Verteilung der Stoffe im Wasser und auf der Haut möglich ist.

### 4.1.1. Vitamine

Als Vitamine kommen wasserlösliche und fettlösliche Substanzen in Betracht. Zu den wasserlöslichen Vertretern zählen insbesondere:
Vitamin C bzw. geeignete Derivate hiervon wie - Palmitat, Calciumascorbat/NatriumAscorbat, Kalium- Ascorbat; ferner die Vitamine der B- Gruppe wie B1 (Thiamin, z. B. Thiaminnitrat), B2 (Riboflavin), B3 (Niacin, Nicotinsäure und Derivate hiervon wie Niacotinsäureamid), B5 (Pantothensäure) wie Calciumpantothenat, B6 (Pyridoxin oder dessen Hydrochlorid), B7 (Vitamin H, Biotin) B9 (Folsäure), B12 (Cobalamin) bzw. geeignete Derivate hiervon oder auch Carotinoide wie Beta/Alpha-Carotin, Beta-Cryptoxanthin, Lutein, Zeaxanthin, Lycopin.

Zu den fettlöslichen Vitaminen gehören vor allem Vitamin E (Tocopherol und Derivate hiervon wie Tocopherylacetat, Tocopherylpalmitat; Tocopherylsuccinat), ferner Vitamin A (Retinol) und Derivate wie Retinylpalmitat, Retinylacetat, Retinyl-proprionat; Vitamin D (Cholecalciferol, Ergocalciferol) oder Vitamin K (Phyllochinon).

Besonders bevorzugt werden Vitamin C, insbesondere -palmitat oder Calciumascorbat, zusammen mit Vitamin - E (insbesondere -acetat, -succinat,) und mit Vitamin B5 und/ oder Vitamin B7 kombiniert. Eine weitere geeignete erfindungsgemäße Mischung ist Vitamin A, insbesondere das -Palmitat, Vitamin- E- Acetat, Beta-Carotin, Folsäure und /oder Vitamin B5.

In einer anderen Ausführungsform werden Vitamin C- z. B. Natriumascorbat zusammen mit Niacin, Biotin und Pantothensäure kombiniert.

Die einzelnen Vitamine bzw. Derivate hiervon werden in den dafür geeigneten Mengen zwischen 0,5 und 3% jeweils, insgesamt in einer Menge von 1 bis 7 Gew.%, bezogen auf die Gesamtgewichtsmenge der Zubereitung eingesetzt.

### 4.1.2. Pflegestoffe

Geeignete Pflegestoffe (wie Rückfetter) sind z. B. essentielle ungesättigte Fettsäuren und deren Ester, z.B. Linol- oder Linolensäure, Glyceryl- Linoleate, Glyceryl- Linolenate. Ein geeignetes Produkt hierzu ist beispielsweise Lamesoft® (Laurinsäuremonoglycerid- Alkylpolyglykosid; INCI-Bezeichnung: Coco Glucoside Glyceryle Oleate).

Eine weitere Gruppe von Pflegestoffen sind Feuchthaltemittel wie Glycerin, Propylenglykol oder Polyethylenglykole, Polypropylenglykol, Butylenglykol, Sorbitol oder Polymere, z.B. Polyquaternium-Typen, Dexpanthenol ((D)-Panthenol), Aminosäuren.

Zu den Hautpflegenden Wirkstoffen gehören z. B. die auch als Vesikelbildner bekannten Phospholipide, wie Lecithin (z.B. Ei- oder Soja-Lecithin), oder Phosphatidylcholin, -serin oder -diethanolamin, Phosphatidylserin Phospatidylinosit, z. B. aus Soja, Raps, Baumwollesamen, Ei, sowie deren Mischungen. Weitere geeignete Pflegestoffe dieser Art sind Sphingolipide (z.B. Ceramide, Cerebroside, Sphingosin, Sphingomyelin), oder auch ethoxylierte Sterole wie Phytosterole (Gemische aus Sistosterol, Camposterol und Stigmasterol), z.B.Ethoxylate hiervon wie PEG-5-Soybean Sterol oder PEG-5 Rapeseed Sterol, =Generol® 122 E5 bzw. Generol® R E5.

Besonders geeignet sind hier Lecithin wie Soja- Eilecithin; Phospholipide wie Phosphatidylcholin, Phosphatidylserin, Phosphatidylethanolamin, Phophatidylglycerol, Phosphatidylinositol, Ganglioside, Cerebroside, Cholesterin und Ceramide, wie z.B. Ceramid-2, -3, -6 sowie Sphingolipide.

Ein weiterer erfindungsgemäßer Pflegestoff ist Harnstoff, der selbst in höheren Mengen wie 10 Gew. % und mehr stabil in die anmeldungsgemäße Mischung eingearbeitet werden kann.

### 4.1.3. Weitere Wirkstoffe: ätherische Öle/Extrakte, anwendungsbezogene Wirkstoffe, feste Kräuter

Weitere Wirkstoffe, welche in der Zubereitung vorliegen können, sind insbesondere ausgewählt aus ätherischen Ölen/Pflanzenextrakten/Kräutern mit den jeweiligen dem Fachmann bekannten Wirkungen wie anregend, beruhigend, heilend, erfrischend, belebend, entspannend, pflegend u.a.m. Auch andere anwendungsbezogene Wirkstoffe wie Durchblutungsfördernde Stoffe, hygienische/dermatologische Wirkstoffe (Antimykotika, Antiseptika, keimreduzierende, antikoagulierende Stoffe u.a.) können hier eingesetzt werden.

### 4.1.4.1.Ätherische Öle/Pflanzenextrakte:

Zu den ätherischen Ölen/Pflanzenextrakten gehören für kosmetische/dermatologische oder ggf. medizinische Badezusätze gebräuchliche ätherische Öle/Extrakte, vorzugsweise ausgewählt aus Ölen/Extrakten aus Khakiblättern, Mango, Feigen, Lavendel, Zedernholz, Lotusblüten, Kamillenblüten, Ylang Ylang, Ginkgo, Kiefernnadel, Zypresse, Orangen, Rosenholz, Pflaumen- Birken- Blätterextrakt, Aloe-Vera-Extrakt, Ringelblumen-, Hibiskus-, Klettenwurzel- Hamamelis-, Wassernabelkraut-, Algen-, Quitten-, Wasserlilien-, Zimtextrakt, Extrakte aus Thymian, Minze, Limetten, Orangen, Grapefruit, Mandarine, Wacholder, Baldrian, Zitronenmelisse, Eukalyptus, Thymian, Palmarosa, Zimt, Rosmarin, Rosenholz, Lemongras, Wildrose, Fichtennadel, Kiefernnadel, Ingwer-, Johannisbeer-, Lindenblüten-, Ringelblumen-, Magnolien-Extrakt, Ananas-, Guave-, Echinacea-, Efeublätter-Extrakt, Heidelbeer-, Kaki-, Melonen- Extrakt u. ä. oder Mischungen hiervon.

Diese Stoffe bzw. Öle werden auf bekannte Weise wie durch Wasserdampfdestillation hergestellt. Hierdurch werden z.B. ätherische Öle aus den genannten Pflanzen erhalten, welche besonders bevorzugt sind. Die Extrakte können durch Lösungsmittelextraktion (mit Alkoholen, Triglyceriden oder Kohlenwasserstoffen, Wasser, Gemischen hiervon) erhalten und als solche dann eingesetzt werden.

Analoge synthetisch hergestellte Substanzen sind beispielsweise Terpene und Terpenoide wie Campher, Menthol, Cineol oder Mischungen hiervon.

### 4.1.4.2. Anwendungsbezogene Wirkstoffe

Hierzu gehören z. B. Durchblutungsfördernde Stoffe (ggf. neben o.g. ätherischen Ölen / Extrakten) wie Nikotinsäurederivate wie Methyl- oder Tocopherylnikotinat, Alpha- und Betahydroxysäuren und deren Derivate, z. B. Glykol-, Äpfel-, Zitronensäure, Weinsäure, Milchsäure, Salicylsäure, Isopropylbenzylsalicylate, C12-13 Alkyl Lactate (Cosmacol^{®} ELI) oder auch antiphlogistische und antibakterielle Substanzen wie Triterpene, z. B. Ursolsäure, Glycyrrhizinsäure oder Glycyrrhetinsäure und deren Derivate, z. B. Stearyl Glycyrrhetinate, Kaliumglycyrrhinat; Pantothensäurederivate, z. B. D-Panthenol, Panthenyltriacetat oder auch Polyphenole, Flavonoide, z. B. Rutin, Ferulasäure und deren Ester oder Isoflavone wie Soja-Isoflavone oder Coenzym Q 10. Auch hygienische/dermatologisch-medizinische Wirkstoffe eignen sich wie z. B. Antimykotika wie Clotrimazol, Ciclopiroxolamin oder Ketoconazol, Antiseptika, Antibiotika, z.B. Gentamicin. Weitere dermatologische Wirkstoffe sind beispielsweise Benzalkoniumchlorid, Chlorhexidin, Dexpanthenol, Allantoin, Bisabolol sowie Lipoproteine z.B. aus Getreide, Mineralstoffe und Spurenelemente wie Kupfer, Zink, bzw. deren Derivate wie Zincidone^{®} (Zink PCA-Pyrrolidoncarbonsäure), Zinkglukonat oder Kupfergluconat.

Es können auch kühlende/beruhigende Wirkstoffe wie Frescolat^{®} ML (Menthyl Lactate) oder Eashave^{®} (Sodium Hyaluronate, Wheat Germ Extrakt, Saccharomyces Cerevisiae Extrakt) inkorporiert werden.

Probiotische Wirkstoffe wie vor allem Lactobacillen, Bifidobacterien oder Mischungen hiervon (wie 10² bis 10⁹ Keime/g Zubereitung) werden anmeldungsgemäß, insbesondere in Verbindung mit nicht ionischen und/oder anionischen Dispergiermitteln, nicht benötigt und sind vorzugsweise daher nicht enthalten.

### 4.1.4.3. Feste Kräuter

Hierzu gehören beispielsweise vor allem Blätter/Blüten/Krautmischungen von Kamille, Lavendel, Heublume, Schachtelhalm, Thymian oder Rosmarin. Derartige feste Kräuter können mit den erfindungsgemäßen Zubereitungen vorzugsweise in perforierten Gebinden wie solchen aus Baumwolle, Kunststoffschläuchen u. ä., welche dann in Sachets gepackt sind, eingesetzt werden. Bei Anwendung können deren Inhaltsstoffe freigesetzt werden, ohne dass die festen unlöslichen Bestandteile in die Badeflotte oder bei der Duschanwendung austreten können (Badetee, Duschkissen).

Bevorzugte Wirkstoffe sind ausgewählt aus ätherischen Ölen, Pflanzenextrakten, Vitaminen, Pflegestoffen, Kräutern oder Kombinationen hiervon.

### 4.2. Zusatzstoffe

Zusatzstoffe werden je nach galenischer Darreichungsform (Salz/Granulat) ausgewählt und in Mengen z. B. zwischen 0 und 15 Gew.%, insbesondere 0,1 bis 10 Gew. %, bevorzugt 0,5 und 7 Gew.%, eingesetzt. Hierzu gehören je nach Konfektionierungsart/Darreichungsform vor allem Antioxidantien, Farb- und Geruchsstoffe oder Mischungen hiervon.

### 4.2.1.Antioxidantien

Hierzu gehören BHT, Butylhydroxyl BHA, Vitamin E, Vitamin C, Propylgallat oder Kombinationen hiervon.

### 4.2.2 Geruchs-/Parfümstoffe

Geruchsstoffe sind z. B. die vorstehend genannten ätherischen Wirkstoffe (Öle, Extrakte) oder synthetische Mittel wie Parfümstoffe. Diese sind dem Fachmann bekannt.

### 4.2.3. Farbstoffe

Farbstoffe sind beispielsweise für den genannten Zweck geeignete zugelassene insbesondere wasserlösliche Farbstoffe, wie z.B. Cochenülerot, Patentblau und Chinolingelb, oder natürliche wasserlösliche Farbstoffe wie Rote Beete- Extrakt, Chlorophyll oder Beta-Carotin. Diese Substanzen sind dem Fachmann bekannt und können je nach beabsichtigtem Zweck (wie Meeresmilieu, grünes Erfrischungsbad, gelbes Fußbad und dgl.) entsprechend ausgewählt werden.

Konservierungsstoffe sind in erfindungsgemäßen Zubereitungen nicht erforderlich. Ebenso sind adsorptive Stoffe wie Aerosile, Kolloide, Dextrine, oder auch Cellulosederivate nicht erforderlich und werden bevorzugt nicht eingesetzt. Dazu gehören vor allem Dextrin, und/oder hochdisperse Kieselsäure oder andere Siliciumdioxid - Produkte, Celluloseether, wie Methylcellulose, Hydroxyethylcellulose, Hydroxypropylcellulose, Guar Gum, Xanthan, Stärke, bzw. Stärke-Derivate wie Reis-, Weizen-, Mais- und Kartoffelstärke, hydrophobisch modifizierte Stärken wie Aluminium Starch Octenylsuccinate, Kolloide wie Eiweißhydrolysate (Proteine) pflanzlichen und/oder tierischen Ursprungs, wie z.B. Getreide- (Weizen-Gerste-Hafer-Mais)proteinhydrolysate und Collagenhydrolysate, (insbesondere mit einer Molekülmasse kleiner/gleich 30 000 Da oder größer 50000 Da); ferner Polyvinylpyrrolidon, leichtes Magnesiumcarbonat oder Mischungen hiervon. Trotz ihres Fehlens werden jedoch stabile Produkte wie beschrieben erhalten.

### Darreichungsformen

Die vorliegenden Zubereitungen liegen in fester einteiliger Form als Granulat/Salz/- Pulver schüttbar, körnig vor. Sie können in geeigneten Mengen portioniert werden und daher anwendungsfreundlich zum Einsatz kommen. Dabei müssen die beiden in unterschiedlichen Aggregatzuständen vorliegenden Grundlagen nicht wie bisher getrennt verpackt oder zuvor emulgativ kombiniert werden. In einer weiteren Ausführungsform werden feste Zubereitungen in Form eines Pulvers/Granulats erhalten aus vorzugsweise 65-80 Gew. % Mineralsalzen, insbesondere ausgewählt aus Natriumchlorid, Natriumsulfat, Totes Meersalz, Meersalz, Steinsalz oder Mischungen hiervon. Weiterhin sind hier vor allem 0-15 Gew.%, vor allem 0,1 bis 15 Gew.%, insbesondere 0,5-10 % Gew.% Wirkstoffe vorhanden, vor allem ausgewählt aus ätherischen Ölen, Pflanzen-Extrakten, Hautpflegenden Stoffen, Vitaminen, Kräutern, oder Mischungen hiervon, sowie 0,1 bis 10, insbesondere bis 7 Gew. % Zusatzstoffe, bevorzugt gewählt aus Parfümstoffen, Farbstoffen, sowie insbesondere 10 -27 Gew. %, vor allem 10-13 Gew.% einer oder mehrerer Lipidkomponenten; 6-15 Gew.%, insbesondere 6-13 Gew. % jeweils eines oder mehrerer TO + TW- Tenside.

Die Angabe ,Gew.%' bezieht sich auf die Gesamtzubereitung, soweit nicht anders angegeben.
Die Zubereitungen werden insbesondere einteilig dargereicht und umfassen die gesamte Mischung, aus der der Anwender die geeignete Menge entnehmen kann. Dies ist überraschenderweise durch einfaches Ausschütten möglich, obwohl die Zubereitung zweiphasig vorliegt. Zusätzlich kann die Zubereitung aufgrund ihrer galenischen Beschaffenheit auch mit der Hand entnommen und direkt vor oder während des Badens zusätzlich angewendet werden. Die Verpackung/Umverpackung kann dabei Hinweise für die jeweilige Anwendungsmenge aufweisen. Es ist insbesondere von Vorteil, eine kleinere Einzelverpackung mit einer geeigneten vordosierten Menge an Zubereitung bereitzustellen wie z. B. kleinere Einzelgebinde für Reisen, Einmalanwendung (Sachets). Das balneologische ölhaltige Salzgranulat oder Salzpulver kann dann als Einmaldosierung leicht angewendet werden. Die Zubereitungen können als Gesamtpackung oder vor allem als Einzelzubereitungen z. B. in Form von Sachets dabei so gestaltet sein, dass die vorgesehene Menge an körniger Zubereitung hieraus entnommen und in der Badeflotte oder auch in sonstigen Badeanwendungen wie direktes Einreiben auf der Haut (Peeling, Body Contour) vor oder während des Bades, oder für ein Fußbad, Armbad, eingesetzt wird. Andererseits können die Gebinde auch netzartig/perforiert gestaltet und in Sachets enthalten sein. Zur Anwendung werden sie den Sachets entnommen und in die Badeflotte eingelegt. Die Erfindung umfasst daher Einmalgebinde bzw. darin enthaltende Zubereitungen, in Form eines geschlossenen oder offenporigen (perforierten) Sachets oder Kissens im Sachet aus Kunststofffolie oder Cellulosefaser, Baumwollgaze, wobei die Zubereitungen in einer Menge von 20 bis 100 g vorliegen.
Alternativ kann mit derartigen Gebinden die Haut auch direkt behandelt werden kann. Dies ist insbesondere geeignet für Anwendungsformen wie festes Kräuterölbadsalz (Badetee), wodurch die Kräuter wieder leicht mit der Packung entfernt werden können, während sich die übrigen Stoffe aus der Zubereitung herauslösen. Netzartige/ perforierte in Sachets enthaltene Kissen oder Schlauchbeutel sind auch insbesondere geeignet für die Anwendung in der Dusche als z. B. Einmalduschsachet als Duschnetz/Duschkissen, oder auch mit besonders hohem hygienischem Anspruch. Schließlich erlaubt die leichte Handhabbarkeit/Schüttbarkeit der erfindungsgemäßen Zubereitung auch eine Abfüllung in größere Behälter, aus denen mehrere Anwendungsdosierungen leicht entnommen werden können.

### Herstellung

Die erfindungsgemäßen Grundlagen bzw. Zubereitungen werden hergestellt durch Herstellung der Vorprodukte in üblichen Apparaturen (Edelstahlkessel mit Rührwerk//Mischer). Dabei werden die (überwiegend) festen Bestandteile (Salzträger, insbesondere feste wasserlösliche Tenside TW, Zusatz- und/oder Wirkstoffe, sofern vorhanden, vorzugsweise oder überwiegend feste Zusatz- und/oder Wirkstoffe) zunächst separat gemischt, ebenso die flüssigen/fließfähigen Komponenten (Lipide, öllösliche Tenside TO, Zusatz- und/oder Wirkstoffe, sofern vorhanden, vorzugsweise oder überwiegend flüssige Zusatz- und/oder Wirkstoffe). Die Vormischung kann bzgl. der flüssigen/fließfähigen Komponenten bei Raumtemperatur bis weniger als 60° C erfolgen, die feste Grundlage wird zweckmäßiger Weise bei Raumtemperatur hergestellt. Sodann werden beide Phasen (bei geeigneter Temperatur) direkt in die gewünschten Abfüllbehältnisse (Sachets, Kissen, Schlauchbeutel als Einmalgebinde oder größere Behältnisse aus für diese Zwecke geeigneten Materialien wie Glas, Kunststoff etc.) ohne vorheriges Zusammenmischen gegeben. Das Abfüllen kann dabei nacheinander, vorzugsweise gleichzeitig in das gewünschte Behältnis erfolgen. Dabei wird das erfindungsgemäße Produkt direkt erhalten. Die feste Zubereitung kann vorzugsweise in Einzelgebinde (Sachets) abgepackt und als Einmalanwendung eingesetzt werden. Im Falle von Kräutern oder zur Anwendung in der Dusche können die Gebinde auch perforiert sein, z. B. in Form von flexiblen Kissen, Schlauchbehältnissen u.ä. und aus geeigneten Materialien wie Baumwolle, Gaze, oder aus anderem für derartige Zwecke geeignetem und bekanntem textilem Kunststoffgewebe bestehen. Diese perforierten Gebinde werden dann zweckmäßiger Weise in Sachets verpackt. Andererseits können auch größere Mengen für mehrere Anwendungen in einer Packung enthalten sein, aus der dann jeweils die gewünschte Menge an Ölbadesalzzubereitung leicht entnommen werden kann, z. B. durch Schütten direkt in die Badeflotte oder in die Hand zum zusätzlichen Auftrag auf die Haut durch Einreiben anlässlich des Badevorgangs.

Es werden insgesamt stabile Produkte auf einfache und sehr kostengünstige Weise ohne großen apparativen Aufwand hergestellt.

Die Erfindung umfasst daher Zubereitungen wie vorstehend beschrieben, welche herstellbar und insbesondere hergestellt sind durch jeweils separates Vormischen einer festen Phase aus Salz, festen wasserlöslichen Tensiden TW und Wirk- und/oder Zusatzstoffen, sofern vorhanden, sowie einer flüssigen Phase aus flüssigen Lipiden (Ölen), öllöslichen Tensiden TO und ggf. vorhandenen Zusatz-und/oder Wirkstoffen und anschließenden, vorzugsweise gleichzeitigen, Eintrag über insbesondere geeignete Abfüllvorrichtungen, beider Phasen ohne vorherige Durchmischung der beiden Phasen fest/flüssig miteinander in geeignete Abfüllbehältnisse, insbesondere Sachets/Kissen.

### Anwendung

Die beschriebenen Zubereitungen können sowohl in der Wanne als auch in der Dusche für Teil- oder Vollbäder für kosmetische, dermatologische oder medizinische Zwecke z.B. zur Herstellung eines Wannenbades eingesetzt werden. Dabei wird vom Anwender die geeignete Menge eingesetzt, im Allgemeinen entsprechend einer auf der die Zubereitung enthaltenden Packung angegebenen Menge. Je nach Anwendungszweck (Indikationsbad) wie Erfrischungsbad, Thermalbad, Entspannungsbad, Fußbad, medizinisches Bad (Erkältung, Rheuma etc.), Kräuterbad, Vitaminbad, Pflegebad, Ätherisches Ölbad, Entspannungsbad oder Kombinationen hiervon können geeignete Wirkstoffe/Wirkstoffkombinationen enthalten sein. Zusätzlich kann die Zubereitung vor allem wegen ihrer besonderen galenischen Form mit der Hand zum zusätzlichen Auftrag auf die Haut anlässlich des Badevorgangs (zum direkten Einreiben auf die Haut vor oder während der Herstellung eines Wannenbades) eingerieben werden. Dadurch kann ein Peeling-Effekt oder eine verbesserte Hautstruktur im Sinne eines Body-Contour- oder Anti-Cellulite-Effektes erzielt werden. Neben der dadurch bedingten Wirkung erfolgt aufgrund der besonderen Kombination der insbesondere natürlichen Grundstoffe auch eine verbesserte Hautwirkung durch die Anwesenheit der flüssigen Lipide in größeren Mengen und die ganzheitliche Hautkontaktierung, ohne dass hierfür besondere Vehikel wie Liposomen oder auch CO₂ (aus einem Brausesatz) vorhanden sein müssten. Ganz besonders geeignet sind die festen Ölbadzubereitungen für die Anwendung im Bad und auch in der Dusche in Form perforierter Sachets, insbesondere Dusch/Wannenkissen (in geeigneten Sachets, da die Zubereitung selbst auch als Auftragsmittel analog einem Schwamm oder Duschnetz verwendet werden kann. Das übrig bleibende Kissen/Netzt und vorzugsweise auch das Sachet sind vor allem aus biologisch verwertbarem Material, so dass die (Dusch/Wannen/-Kräuter)-Kissen/-Netz-Zubereitung den heute hohen Ansprüchen sowohl in hygienischer als auch ökologischer Sicht gerecht werden kann.

Die erfindungsgemäße Zubereitung ist daher auch einsatzflexibel.

Die Dauer der Anwendung richtet sich nach dem jeweiligen Ziel, wie z. B. 10 bis 30 Minuten Wannebad / Fußbad, einige Minuten Duschbad. In der Wanne sollte die Temperatur des Wassers zwischen 33°C und 42°C betragen, in der Dusche oder beim Fußbad wird sie den Verhältnissen angepasst. Die Zubereitungen sind so konzipiert, dass nach Beendigung der Anwendung keine besonderen Maßnahmen zum Entfernen eventueller Rückstände zu ergreifen sind. Nachspülen mit Wasser und/oder Abwischen, sofern gewünscht, kann jedoch erfolgen.

### Beispiele

Die Erfindung wird anhand der nachfolgenden Beispiele 1-9 näher erläutert, welche lediglich erläuternd, nicht jedoch einschränkend sind. Die Zubereitungen 1-9 wurden dabei wie oben beschrieben hergestellt. Die Beispiele 10-14 stellen Vergleichszubereitungen dar. Beispiel 15 umfasst anwendungsbezogene Hautfettmesstests hiermit.

**Beispiel 1: Pflegeölbadsalz**

| Inhaltsstoff | Gew.% |
|---|---|
| Meersalz | 80 |
| Natriumlaurylsulfat | 2 |
| D-Panthenol | 0,5 |
| Parfümöl | 1 |
| FS Gelborange | 0,1 |
| Calendulaextrakt | 1 |
| Sojaöl | 6 |
| Mandelöl | 6 |
| Laureth-4 | 3 |
| Aloe Vera | 0,4 |
| | 100 |

**Beispiel 2 Pflegeölbadesalz**

| Inhaltsstoff | Gew.% |
|---|---|
| Sole Salz | 70 |
| Natrium Methyl Oleoyltaurate | 3 |
| Orangenblütenöl | 1 |
| Mandelöl | 1 |
| Cochenille rot | 0,2 |
| MIPA Laureth Sulfate | 4 |
| Laureth-4 | 4 |
| Cocamide DEA | 3 |
| Erdnussöl | 10 |
| Ringelblumenextrakt | 2 |
| Parfümöl | 1 |
| Vitamin-E-Acetat | 0,8 |
| | 100 |

**Beispiel 3 Multivitaminölbadesalz**

| Inhaltsstoff | Gew.% |
|---|---|
| totes Meersalz | 65 |
| Harnstoff | 10 |
| Ascorbinsäure | 2 |
| Nikotinsäure | 1 |
| Thiaminhydrochlorid | 0,5 |
| Pyridoxinhydrochlorid | 0,5 |
| Rizinussöl | 2 |
| Tween 20 | 2 |
| Sojaöl | 8 |
| Avocadoöl | 3 |
| Vitamin A-Acetat | 1 |
| Parfümöl | 1 |
| Limettenöl | 1 |
| Vitamin-F (enthält Linolsr.Linolensr.Ölsr. Palmitinsr.Stearinsr.) | 1 |
| Disodiumlaurylsulfosuccinat | 1 |
| | 100 |

**Beispiel 4 Pflegeölbadesalz**

| Inhaltsstoff | Gew.% |
|---|---|
| Steinsalz | 50 |
| Thermalbadesalz (K,Mg,Ca) | 25 |
| Disodium Lauryl Sulfosuccinate | 2 |
| Patentblau | 0,1 |
| Vanilleextrakt | 1 |
| Parfümöl | 1 |
| Arganöl | 2 |
| Lecithin | 2 |
| Sonnenblumenöl | 9 |
| Laureth-2 | 3 |
| Vitamin E-Acetat | 1 |
| Feigenextrakt | 2 |
| Kokosöl | 1,9 |
| | 100 |

**Beispiel 5: PflegeÖlbadesalz-Kräuterkissen**

| Inhaltsstoff | Gew.% |
|---|---|
| Kamille | 3 |
| Schachtelhalm | 3 |
| Heublumen | 4 |
| Siedesalz | 65 |
| Harnstoff | 7 |
| Ammonium Laurylsulfate | 2 |
| Lemongrasöl | 1 |
| Citronellöl | 1 |
| Olivenöl | 6 |
| Weizenkeimöl | 4 |
| Kokosfettsäurediethanolamid | 2 |
| Aprikosenkernöl | 2 |
| | 100 |

**Beispiel 6 Fußölbadesalz**

| Inhaltsstoff | Gew.% |
|---|---|
| Siedesalz extrafein | 53 |
| Magnesiumchlorid | 2,5 |
| Natriumsulfat | 20 |
| Arnikaextrakt | 0,2 |
| Orangenöl | 0,2 |
| Eucalyptusöl | 0,2 |
| Rosmarinöl | 0,2 |
| Chinolingelb | 0,1 |
| MIPA Laureth Sulfate | 4 |
| Laureth-4 | 4 |
| Cocamide DEA | 3 |
| Sojaöl | 8 |
| Jojobaöl | 1 |
| Mandelöl | 1 |
| Laureth-2 | 0,5 |
| Panthenol | 1,1 |
| Parfümöl | 1 |
| | 100 |

**Beispiel 7: Pflegeölbadesalz mit Harnstoff**

| Inhaltsstoff | Gew.% |
|---|---|
| Meersalz | 74 |
| Harnstoff | 8 |
| Natriumlaurylsulfat | 2 |
| D-Panthenol | 0,5 |
| Parfümöl | 1 |
| FS Gelborange | 0,1 |
| Calendulaextrakt | 1 |
| Sojaöl | 5 |
| Mandelöl | 5 |
| Laureth-4 | 3 |
| Aloe Vera | 0,4 |
| | 100 |

**Beispiel 8: Pflegeölbadesalz**

| Inhaltsstoff | Gew.% |
|---|---|
| Totes Meersalz | 75 |
| Natriumoleyltaurat | 1 |
| Pfirsischkernöl | 22 |
| Tween 20 | 1 |
| Parfümöl | 1 |
| | 100 |

**Beispiel 9 Pflegeölbadesalz mit Harnstoff**

| Inhaltsstoff | Gew.% |
|---|---|
| Sole Salz | 68 |
| Harnstoff | 8 |
| Natriumoleylsuccinat | 1 |
| Traubenkernöl | 20 |
| Laureth 4 | 1 |
| Parfümöl | 2 |
| | 100 |

### Vergleichsbeispiele 10-14

Zum Vergleich mit erfindungsgemäßen Zubereitungen im Sebumetertest zur Ermittlung des Hautfettgehaltes nach dem Bade (Anwendungstestbeispiel 15) wurden die folgenden Zubereitungen gemäß Stand der Technik eingesetzt.

**Beispiel 10 (Vergleich 1) : Ölbad mit hohem Ölanteil**

| Inhaltsstoff | Gew.% |
|---|---|
| Sojaöl | 95 |
| Laureth-3 | 3 |
| Parfümöl | 2 |
| | 100 |

**Beispiel 11 (Vergleich 2): Ölschaumbad**

| Inhaltsstoff | Einsatzkonz. in % |
|---|---|
| Mandelöl | 20 |
| MIPA Laurethsulfat | 60 |
| Tween 20 | 10 |
| Eukalyptusöl | 7 |
| Parfümöl | 3 |
| | 100 |

**Bespiel 12: (Vergleich 3): Badekonzentrat**

| Inhaltsstoff | Gew.% |
|---|---|
| Macademianussöl | 1 |
| Natriumlaurylethersulfat | 14 |
| Laureth 3 | 15 |
| Lavendelöl | 8 |
| Benzylalkohol | 1 |
| Parfümöl | 2 |
| Wasser | 59 |
| | 100 |

**Beispiel 13: (Vergleich 4): Handelsware Kinderölbad (reines Ölbad)**

| Inhaltsstoff | Gew.% |
|---|---|
| Mandelöl | k.A. |
| Olivenöl | k.A |
| Jojobaöl | k.A. |
| Weizenkeimöl | k.A. |
| Parfümöl | k.A. |
| | 100 |

**Beispiel 14 (Vergleich 5): Handelsware Öltensidbad mit natürlichen Ölen**

| Inhaltsstoff | Gew.% |
|---|---|
| Mandelöl | k.A. |
| Rizinusöl | k.A |
| MIPA Laurethsulfat | k.A. |
| Wildrosenöl | k.A. |
| Lavendelöl | k.A. |
| Parfümöl | k.A. |
| | 100 |

| | |
|---|---|
| k.A.: Keine prozentuale Angabe des Herstellers | |

### Anwendungstestbeispiel 15

Im Folgenden wurden erfindungsgemäße Zubereitungen gemäß den Beispielen 8, 9 (Erf.) im Vergleich zu den oben beschriebenen bekannten Ölbadezubereitungen der Vergleichsbeispiele 10-14 im Hinblick auf die Fettung der Haut nach dem Bade getestet. Dazu wurde die Talgsekretion gemessen. Als Meßmethode wurde das Meßprinzip Sebumeter angewandt. Dabei handelt es sich um eine photometrische Methode (Fettfleckphotometer) zur Erfassung des Hautfettes, nicht jedoch der Hautfeuchtigkeit. Die Messung mittels Fettfleckphotometer erfolgt über eine Meßkassette, die ein 0,1mm starkes mattiertes Kunststoffband aufweist. Dieses wird auf die zu messende Position aufgedrückt. Die Messfläche beträgt 64 mm². Die Meßzeit beläuft sich auf 30 Sekunden pro Messung. Gemessen wird die Transparenz der Folie mittels einer Photozelle. Die Änderung der Lichtdurchlässigkeit ist der Maßstab für den Oberflächen-Talg(Fett)-gehalt.

### Versuchsanordnung:

Die Testzubereitungsproben werden so in eine 10l Testwanne eingewogen, daß pro 10l Badinhalt immer die gleiche Menge an Lipid im Wasser vorhanden ist. Die Badetemperatur liegt dabei zwischen ca. 40°C und ca. 44°C.

Der nicht eingecremte Unterarm der Testperson wird unter leichter Bewegung 5 Minuten komplett untergetaucht. Nach Ende der Badedauer wird der Arm an der Luft getrocknet, und nicht mit einem Handtuch abgewischt. Die Sebumetermessung wird nach 10 Minuten wie oben erläutert durchgeführt, indem die Meßkassette mit der Kunststofffolie auf das zu messende Armareal angelegt und nach 30 Sekunden in das Sebumeter-Messgerät eingelegt wird. Dieses zeigt die Meßdaten automatisch an. Laut Hersteller (Sebumeter SM 810) liegen die Werte für normale Haut (Arm, Hand, Beine) bei mehr als 6 µg Sebum/cm² . Für trockene Haut beträgt dieser Wert 0 bis 6 µg Sebum/cm².

Die nach der oben beschriebenen Sebumetermethode erhaltenen Meßwerte der Zubereitungen gemäß den Beispielen 8, 9 (Erfindung) und den Vergleichszubereitungen 10-14 sind in Tabelle 1 zusammengefasst, wobei die Meßwerte jeweils Mittelwerte aus drei Einzelmessungen am gleichen Probanden sind.

**Tabelle 1: Meßergebnisse:**

| Bsp. Nr.: | Hautfettmessung Arm / Fettanteil µg/qcm |
|---|---|
| 8 Erfindung | 14,2 |
| 9 Erfindung | 14,5 |
| 10 Vergleich 1 | 9,5 |
| 11 Vergleich 2 | 2,5 |
| 12 Vergleich 3 | 3 |
| 13 Vergleich 4 | 4 |
| 14 Vergleich 5 | 5 |

Die Ergebnisse sind in Abbildung 1 nochmals graphisch dargestellt. Wie hieraus ersichtlich, sind mit den erfindungsgemäßen festen Ölbadesalzen nicht nur verbesserte Werte bzgl. des Lipidstatus bezogen auf bekannte Ölbäder (Vergleichsbeispiele 10,13) mit insbesondere höherem Ölanteil als in erfindungsgemäßen Zubereitungen erzielbar, sondern darüber hinaus noch unerwarteterweise verbesserte Werte auch gegenüber weiteren ähnlichen Produkten (Vergleichsbeispiele 11,12,14) .

## Patentansprüche

1. Feste, im Wesentlichen wasserfreie Ölbadesalzzubereitung, **dadurch gekennzeichnet, dass** sie aus mehr als 10 Gew.% bis 35 Gew.% einer flüssigen Ölgrundlage sowie 65 bis weniger als 90 Gew.% eines festen Trägers, jeweils bezogen auf die Gesamtzubereitung, besteht, wobei die Ölgrundlage ein oder mehrere flüssige Lipide, ausgewählt aus Olivenöl, Sonnenblumen-, Sojaöl, Pfirsichkern-, Aprikosenkern-, Traubenkern-, Ricinus-, Erdnuß-, Mandel-, Weizenkeim-, Sesam-Distel-, Avocadoöl, Shea Butter oder Illipé Butter; Jojobaöl, Kokosnussöl, Borretschöl, Maiskeimöl, Walnussöl, Palmöl, Macadamianussöl, Palmkernöl, Haselnussöl, Hagebuttenkernöl, Baumwollsamenöl, Papayaöl oder Mischungen hiervon, oder Mischungen hiervon mit einem oder mehreren flüssigen C₈₋₂₂-Fettalkoholen, weiterhin ein oder mehrere öllösliche Tenside (TO) sowie ggf. Zusatz/Wirkstoffe umfasst, und wobei der feste Träger auf einer Salzgrundlage basiert und weiterhin ein oder mehrere wasserlösliche Tenside (TW) und ggf. Zusatz- und/oder Wirkstoffe umfasst, mit der Maßgabe, dass in der Gesamt(Ölbadesalz)-zubereitung höchstens 80 Gew.% Salz sowie mindestens 10 Gew.% flüssiges Lipid umfasst sind, und die Gesamtmenge an flüssigem Lipid(en) + Tensiden mehr als 15 Gew.% beträgt, und wobei die Zubereitung ohne Aufeinanderauftragung, Mischung der Phasen als 2-phasiges schüttbares Produkt aus flüssiger Ölphase neben der nicht damit vermischten festen Trägerphase vorliegt.

2. Zubereitung nach Anspruch 1, worin das Tensid TW im festen Träger dort in einer Menge von 0,5 bis 8 Gew.% enthalten und ausgewählt ist aus wasserlöslichen anionischen Tensiden der Gruppe, umfassend C₈₋₂₂- Alkylsulfate, C₈₋₂₂-Alkylethersulfate bzw. deren Alkalisalze, (Alkyl)- C₁₂-C₂₂ -Sulfo-Succinate, (Alkyl)- C₁₂ -C₂₂-Fettalkohol-Aminosäureester-Verbindungen, wobei die Aminosäuren ausgewählt sind aus Glycin, Taurin; und worin das Tensid TO in der Ölgrundlage dort in einer Menge von 1 bis 20 Gew. %, insbesondere 2 bis 15 Gew.% enthalten und ausgewählt ist aus öllöslichen nicht ionischen Tensiden der Gruppe, umfassend polyoxylierte C₈₋₂₂-Fettalkoholether und C₁₂₋₂₂- Fettsäureester mit einem Ethoxylierungsgrad und/oder Propoxylierungsgrad (EO/PO-Grad) von 1-18, vorzugsweise 1 bis 5, Zuckerestern, Zuckerether von C₁₂₋₂₂- Fettsäuren, C₁₂₋₂₂- Fettsäureglycerylestern bzw. C₈₋₂₂-Fettalkoholen, oder Mischungen hiervon.

3. Zubereitung nach einem der Ansprüche 1 oder 2, **dadurch gekennzeichnet, dass** das oder die flüssigen Lipide ausgewählt sind aus Aprikosenkernöl, Avocadoöl, Borretschsamenöl, Distelöl, Erdnussöl, Haselnussöl, Kürbiskernöl, Maiskeimöl, Mandelöl, Marulaöl, Macademianußöl, Mohnöl, Olivenöl, Palmöl, Pinienkernöl, Reiskeimöl, Sanddornöl, Sesamöl, Sojaöl, Sonnenblumenöl, Traubenkernöl, Walnussöl, Weizenkeimöl, Erdnußöl, Distelöl, oder Mischungen dieser Öle.

4. Zubereitung nach einem der Ansprüche 1 bis 3, **dadurch gekennzeichnet, dass** der feste Träger auf einer Salzgrundlage aus Mineralsalzen aus den Kationen Natrium, Kalium, Magnesium, Calcium, Eisen, Ammonium, Lithium, Aluminium, Strontium oder Mangan und den Anionen Chlorid, Fluorid, Iodid, Bromid, Thiosulfat, Sulfat oder Nitrat, oder aus Meersalz, Pfannensiedesalz, Totes Meersalz, Steinsalz, Siedesalz oder geeigneten Mischungen hiervon beruht.

5. Zubereitung nach einem der Ansprüche 1 bis 4, **dadurch gekennzeichnet, dass** Wirkstoffe enthalten sind, ausgewählt aus Vitaminen (Vitaminbad /Dusche), Kräutern (Kräuterbad/Dusche), ätherischen Ölen, Pflanzen-Extrakten, Hautpflegenden Wirkstoffen (Indikationsbad/Duschen), oder Kombinationen hiervon.

6. Zubereitung nach einem der Ansprüche 1 bis 5, **dadurch gekennzeichnet, dass** sie frei von festen Lipiden ist.

7. Zubereitung nach Anspruch 1-3 oder 5-6, **dadurch gekennzeichnet, dass** sie frei ist von Brausesalz (Carbonaten + Säure) .

8. Zubereitung nach einem der Ansprüche 1 bis 7, herstellbar durch jeweils separates Vormischen einer festen Phase aus Salz, festen wasserlöslichen Tensiden TW und Wirk-und/oder Zusatzstoffen, sofern vorhanden, sowie einer flüssigen Phase aus flüssigen Lipiden (Ölen), öllöslichen Tensiden TO und ggf. vorhandenen Zusatz-und/oder Wirkstoffen und anschließenden Eintrag beider Phasen ohne vorherige Durchmischung in geeignete Abfüllbehältnisse.

9. Zubereitung gemäß einem der Ansprüche 1 bis 8, **dadurch gekennzeichnet, dass** sie als Einmalgebinde in Form eines geschlossenen oder offenporigen (perforierten) Sachets oder Kissens im Sachet aus Kunststofffolie oder Cellulosefaser, Baumwollgaze, in einer Menge von 20 bis 100 g vorliegt.

10. Verfahren zur Herstellung einer festen 2-phasigen Ölbadesalz-Zubereitung gemäß einem der Ansprüche 1 bis 9, **dadurch gekennzeichnet, dass** eine feste Phase aus Salz, insbesondere festen wasserlöslichen Tensiden TW sowie Wirk-und/ oder Zusatzstoffen, sofern vorhanden, und eine flüssige Phase aus flüssigen Lipiden (Ölen), öllöslichen Tensiden TO sowie ggf. vorhandenen Zusatz-und/oder Wirkstoffen jeweils separat vorgemischt wird, sodann ohne Durchmischung beider Phasen diese sogleich in geeignete Abfüllbehältnisse gleichzeitig mittels geeigneter Abfüllvorrichtungen eingebracht werden.

11. kosmetische Verwendung einer Zubereitung gemäß einem der Ansprüche 1 bis 10 als Zusatz für Voll- oder Teilbäder, für die Wanne, Dusche, Fußbad oder zum direkten Einreiben auf die Haut vor oder während der Herstellung eines Wannenbades.

12. Verwendung gemäß Anspruch 11 in Form einer Gesamtzubereitung für die Mehrfachanwendung, einer Einzelzubereitung, eines Duschkissensachets, eines Sachets, eines festen Fußölbadesalzes, eines Pflegeölbadesalzsachets.

13. Nicht therapeutische kosmetische Verwendung gemäß einem der Ansprüche 11 oder 12 für ein Wannen- oder Duschbad in Form eines Vitaminölbadesalzes, eines Pflegeölbadesalzes, eines Kräuterölbadesalzes, eines Ätherischen Ölbadesalzes, oder eines Ölbadesalzes mit Wirkstoffkombinationen, zur Erzielung eines hautstrukturierenden, beruhigenden, entspannenden, durchblutungsfördernden, pflegenden Effekts, eines Peelingeffekts oder Kombinationen hiervon.

## Claims

1. Solid, essentially anhydrous oil bath salt preparation, **characterised in that** it consists of more than 10 wt % to 35 wt % of a liquid oil basis as well as 65 to less than 90 wt % of a solid carrier, each relating to the total preparation, wherein the oil base comprises one or more liquid lipids, selected from olive oil, sunflower oil, soya oil, peach kernel oil, apricot kernel oil, grapeseed oil, castor oil, peanut oil, almond oil, wheat germ oil, sesame oil, safflower oil, avocado oil, Shea butter or Illipé butter; jojoba oil, coconut oil, borage oil, maize germ oil, walnut oil, palm oil, macadamia nut oil, palm kernel oil, hazelnut oil, rosebush oil, cotton seed oil, papaya oil or mixtures thereof, or mixtures thereof with one or more liquid C₈₋₂₂fatty alcohols, also one or more oil-soluble surfactants (TO) as well as optional additives/active substances, and wherein the solid carrier is based on a salt basis and also comprises one or more water-soluble surfactants (TW) and optionally additives and/or active substances, with the proviso that in the total (oil bath salt) preparation at most 80 wt % salt and at least 10 wt % liquid lipid are comprised, and the total amount of liquid lipid(s) + surfactants is more than 15 wt %, and wherein the preparation without application on top of one another, mixing of the phases, exists as a 2-phase bulk product of a liquid oil phase in addition to the solid carrier phase that has not been blended with it.

2. Preparation according to claim 1, in which the surfactant TW is comprised in the solid carrier in an amount of 0.5 to 8 wt % and is selected from water-soluble anionic surfactants from the group comprising C₈₋₂₂ alkyl sulfates, C₈₋₂₂ alkyl ether sulfates or their alkali metal salts, (alkyl)-C₁₂-C₂₂ sulfosuccinates, (alkyl)-C₁₂-C₂₂ fatty alcohol-amino acid ester compounds, wherein the amino acids are selected from glycine, taurine; and in which the surfactant TO is comprised in the oil basis in an amount of 1 to 20 wt %, in particular 2 to 15 wt %, and selected from oil-soluble non-ionic surfactants from the group comprising polyoxylated C₈₋₂₂ fatty alcohol ethers and C₁₂₋₂₂ fatty acid esters with an ethoxylation degree and/or propoxylation degree (EO/PO degree) of 1 - 18, preferably 1 - 5, sugar esters, sugar ethers of C₁₂₋₂₂ fatty acids, C₁₂₋₂₂ fatty acid glyceryl esters or C₈₋₂₂ fatty alcohols, or mixtures thereof.

3. Preparation according to one of claims 1 or 2, **characterised in that** the liquid lipid or lipids are selected from apricot kernel oil, avocado oil, borage oil, safflower oil, peanut oil, hazelnut oil, pumpkin seed oil, maize germ oil, almond oil, marula oil, macadamia nut oil, poppy seed oil, olive oil, palm oil, pine nut oil, rice germ oil, sea buckthorn oil, sesame oil, soya oil, sunflower oil, grapeseed oil, walnut oil, wheat germ oil, peanut oil, safflower oil, or mixtures of these oils.

4. Preparation according to one of claims 1 to 3, **characterised in that** the solid carrier is based on a salt basis of mineral salts from the cations sodium, potassium, magnesium, calcium, iron, ammonium, lithium, aluminium, strontium or manganese and from the anions chloride, fluoride, iodide, bromide, thiosulfate, sulfate or nitrate, or from sea salt, panned evaporated salt, Dead Sea salt, rock salt, evaporated salt or suitable mixtures thereof.

5. Preparation according to one of claims 1 to 4, **characterised in that** active substances are comprised, selected from vitamins (vitamin bath/shower), herbs (herbal bath/shower), ethereal oils, plant extracts, skin-care active substances (indication bath/shower), or combinations thereof.

6. Preparation according to one of claims 1 to 5, **characterised in that** it is free of solid lipids.

7. Preparation according to claim 1 - 3 or 5 - 6, **characterised in that** it is free of effervescent salt (carbonates + acid).

8. Preparation according to one of claims 1 to 7, producible by separately premixing a solid phase of salt, solid water-soluble surfactants TW and active substances and/or additives, when present, as well as a liquid phase of liquid lipids (oils), oil-soluble surfactants TO and optionally present additives and/or active substances, respectively, and subsequently filling both phases into suitable filling receptacles without prior stirring.

9. Preparation according to one of claims 1 to 8, **characterised in that** it is present as a single-use package in the form of a closed or open-pore (perforated) sachet or cushion in a sachet made of plastic film or cellulose fibre, cotton gauze, in an amount of 20 to 100 g.

10. Process for manufacturing a solid 2-phase oil bath salt preparation according to one of claims 1 to 9, **characterised in that** a solid phase of salt, in particular solid water-soluble surfactants TW as well as active substances and/or additives, when present, and a liquid phase of liquid lipids (oils), oil-soluble surfactants TO as well as optionally present additives and/or active substances, respectively, are separately premixed, then without stirring, both phases are immediately simultaneously introduced into suitable filling receptacles by means of suitable filling devices.

11. Cosmetic use of a preparation according to one of claims 1 to 10 as an additive for complete or partial baths, for tubs, showers, footbaths or for rubbing directly onto the skin before or during the preparation of a bath.

12. Use according to claim 11 in the form of a complete preparation for repeated use, of a single preparation, of a shower cushion sachet, of a sachet, of a solid foot oil bath salt, of a care oil bath salt sachet.

13. Non-therapeutic cosmetic use according to one of claims 11 or 12 for a bath or shower in the form of a vitamin oil bath salt, of a care oil bath salt, of a herbal oil bath salt, of an ethereal oil bath salt, or of an oil bath salt with combinations of active substances, in order to obtain a skin-structuring, calming, relaxing, circulation enhancing, care effect, a peeling effect or combinations thereof.

## Revendications

1. Préparation solide de sels de bain huileux, sensiblement exempte d'eau, **caractérisée en ce qu'**elle est constituée à plus de 10 % en poids et jusqu'à 35 % en poids d'une base huileuse liquide ainsi qu'à 65 % à moins de 90 % en poids d'un support solide, ces indications se rapportant à la préparation dans sa totalité, ladite base huileuse comprenant un ou plusieurs lipides liquides choisis parmi les huiles d'olive, de tournesol, de soja, de noyau de pêche, de noyau d'abricot, de pépin de raisin, de ricin, d'arachide, d'amande, de germe de blé, de sésame, de carthame, d'avocat, le beurre de karité, le beurre d'illipé ; les huiles de jojoba, de coco, de bourrache, de maïs, de noix, de palme, de noix de macadamia, de palmiste, de noisette, de rose musquée, de coton, de papaye, ou parmi leurs mélanges ou les mélanges qu'ils forment avec un ou plusieurs alcools gras liquides en C₈₋₂₂ et, en outre, un ou plusieurs agents tensioactifs solubles dans l'huile (TO) ainsi que, optionnellement, des additifs / principes actifs, et ledit support solide étant élaboré à partir d'une base saline tout en comprenant, en outre, un ou plusieurs agents tensioactifs hydrosolubles (TW) et, optionnellement, des additifs et/ou des principes actifs, à condition que la préparation (de sels de bain huileux) comprenne, dans sa totalité, au maximum 80 % en poids de sel ainsi qu'au moins 10 % en poids de lipide liquide, la quantité totale de lipide(s) liquide(s) + agents tensioactifs étant supérieure à 15 % en poids, et ladite préparation se présentant, hors mélange de ses phases ou application l'une sur l'autre, sous forme d'un produit à deux phases lequel est susceptible d'être versé et lequel est constitué de ladite phase huileuse liquide et de ladite phase support solide, non mélangée à cette dernière.

2. Préparation selon la revendication 1, dans laquelle l'agent tensioactif TW est contenu dans le support solide dans une quantité comprise entre 0,5 et 8 % en poids tout en étant choisi parmi les agents tensioactifs anioniques hydrosolubles appartenant au groupe comprenant des alkylsulfates en C₈₋₂₂, des alkyléthersulfates en C₈₋₂₂ ou leurs sels alcalins, des (alkyl)-sulfosuccinates en C₁₂ à C₂₂, des (alkyl)-composés issus de l'estérification d'acides aminés avec des alcools gras en C₁₂ à C₂₂, les acides aminés étant choisis parmi la glycine, la taurine; et dans laquelle l'agent tensioactif TO est contenu dans la base huileuse dans une quantité comprise entre 1 et 20 % en poids, notamment entre 2 et 15 % en poids, tout en étant choisi parmi les agents tensioactifs non ioniques solubles dans l'huile appartenant au groupe comprenant les éthers d'alcools gras en C₈₋₂₂ et esters d'alcools gras en C₁₂₋₂₂ polyoxylés ayant un degré d'éthoxylation et/ou de propoxylation (degré EO/PO) compris entre 1 et 18, préférentiellement entre 1 et 5, les esters de sucres, les éthers de sucres d'acides gras en C₁₂₋₂₂, les esters glycéryliques d'acides gras en C₁₂₋₂₂ ou les alcools gras en C₈₋₂₂, ou leurs mélanges.

3. Préparation selon l'une des revendications 1 ou 2, **caractérisée en ce que** la ou les lipides liquides sont choisis parmi les huiles de noyau d'abricot, d'avocat, de graines de bourrache, de carthame, d'arachide, de noisette, de pépin de courge, de maïs, d'amande, de marula, de noix de macadamia, d'oeillette, d'olive, de palme, de pignon de pin, de germe de riz, d'argousier, de sésame, de soja, de tournesol, de pépin de raisin, de noix, de germe de blé, d'arachide, de carthame ou parmi les mélanges de ces huiles.

4. Préparation selon l'une des revendications 1 à 3, **caractérisée en ce que** le support solide est élaboré à partir d'une base saline constituée de sels minéraux formés à partir des cations sodium, potassium, magnésium, calcium, fer, ammonium, lithium, aluminium, strontium ou manganèse et des anions chlorure, fluorure, iodure, bromure, thiosulfate, sulfate ou nitrate, ou constituée de sel marin, de sel raffiné à la poêle, de sel de la mer morte, de sel gemme, de sel raffiné ou de mélanges appropriés de ceux-ci.

5. Préparation selon l'une des revendications 1 à 4, **caractérisée en ce qu'**elle contient des principes actifs choisies parmi les vitamines (préparation vitaminée pour bain/douche), les herbes (préparation à base d'herbes pour bain/douche), les huiles essentielles, les extraits végétaux, les principes actifs pour soins cutanés (préparation pour bain/douche soignant certaines indications) ou parmi leurs combinaisons.

6. Préparation selon l'une des revendications 1 à 5, **caractérisée en ce qu'**elle est exempte de lipides solides.

7. Préparation selon les revendications 1 à 3 ou 5 à 6, **caractérisée en ce qu'**elle est exempte de sels à effet effervescent (carbonates + acide).

8. Préparation selon l'une des revendications 1 à 7, pouvant être fabriquée en réalisant par pré-mélange, séparément, une phase solide constituée de sel, d'agents tensioactifs hydrosolubles TW et, le cas échéant, de principes actifs et/ou d'additifs ainsi qu'une phase liquide constituée de lipides liquides (d'huiles), d'agents tensioactifs solubles dans l'huile (TO) et, le cas échéant, d'additifs et/ou de principes actifs, les deux phases étant ensuite introduites dans des contenants appropriés sans les mélanger préalablement.

9. Préparation selon l'une des revendications 1 à 8, **caractérisée en ce qu'**elle correspond à un emballage à usage unique lequel en contient une quantité comprise entre 20 et 100 g et lequel se présente sous forme d'un sachet hermétique ou poreux (perforé) ou d'un coussin au sein d'un tel sachet en feuille de matière plastique ou en fibres de cellulose, en gaze de coton.

10. Procédé de fabrication d'une préparation de sels de bain huileux à deux phases selon l'une des revendications 1 à 9, **caractérisé en ce que** l'on réalise séparément, par pré-mélange, une phase solide constituée de sel, d'agents tensioactifs hydrosolubles TW, notamment solides, ainsi que de principes actifs et/ou d'additifs, et une phase liquide constituée de lipides liquides (d'huiles), d'agents tensioactifs solubles dans l'huile TO et, le cas échéant d'additifs et/ou de principes actifs, pour ensuite introduire chacune des deux phases au moyen de dispositifs de remplissage appropriés dans des contenants appropriés, simultanément mais sans les mélanger.

11. Utilisation cosmétique d'une préparation selon l'une des revendications 1 à 10, en tant que produit pour bains complets ou partiels, pour la baignoire, la douche, le bain de pied, ou à étaler directement sur la peau avant ou pendant un bain.

12. Utilisation selon la revendication 11 sous forme d'une préparation entière destinée à l'usage multiple, d'une préparation à usage unique, d'un sachet-coussin pour la douche, d'un sachet, d'un sel de bain huileux pour pieds sous forme solide, d'un sachet de sel de bain huileux destiné à des soins.

13. Utilisation cosmétique non-thérapeutique selon l'une des revendications 11 ou 12 pour un bain en baignoire ou pour la douche, sous forme d'un sel de bain huileux vitaminé, d'un sel de bain huileux destiné à des soins, d'un sel de bain huileux aux herbes, d'un sel de bain aux huiles essentielles, ou d'un sels de bain huileux comportant une combinaison de principes actifs, pour obtenir un effet de structuration de la peau, d'apaisement, de relaxation, de stimulation de la circulation sanguine, de soins, un effet d'exfoliation ou des combinaisons de ceux-ci.
